# EUROPÄISCHE PATENTSCHRIFT

(11) **EP 1 229 936 B1**
(45) Veröffentlichungstag und Bekanntmachung des Hinweises auf die Patenterteilung: **19.05.2004**
(21) Anmeldenummer: 00987250.8
(22) Anmeldetag: 15.11.2000
(51) Int. Cl.: A61K 39/395, C07K 16/06, A61K 39/39, A61P 35/00, C07K 1/22

(54) **VERWENDUNG VON ANTI-IDIOTYPISCHEN ANTIKÖRPERN ALS IMPFSTOFFE GEGEN KREBS**
USE OF ANTI-IDIOTYPICAL ANTIBODIES AS VACCINES AGAINST CANCER
UTILISATION D'ANTICORPS ANTI-IDIOTYPIQUES EN TANT QUE VACCINS CONTRE LE CANCER

(30) Priorität: 16.11.1999 AT 192799
(43) Veröffentlichungstag der Anmeldung: 14.08.2002
(73) Patentinhaber: Igeneon Krebs-Immuntherapie Forschungs- und Entwicklungs-AG, 1230 Wien (AT)
(72) Erfinder: LOIBNER, Hans, A-1238 Wien (AT); ECKERT, Helmut, CH-4104 Oberwil (CH); HIMMLER, Gottfried, A-1180 Wien (AT)
(74) Vertreter: VOSSIUS & PARTNER
(86) Internationale Anmeldenummer: PCT/EP2000/011306
(87) Internationale Veröffentlichungsnummer: WO 2001/035989

(56) Entgegenhaltungen:
- WO-A-84/02848
- WO-A-89/11296
- WO-A-91/13632
- US-A- 5 679 356
- LOSMAN M J ET AL: "Human response against NP-4, a mouse antibody to carcinoembryonic antigen: human anti-idiotype antibodies mimic an epitope on the tumor antigen" PROCEEDINGS OF THE NATIONAL ACADEMY OF SCIENCES OF USA,NATIONAL ACADEMY OF SCIENCE. WASHINGTON,US, Bd. 88, Nr. 8, 15. April 1991 (1991-04-15), Seiten 3421-3425, XP002147167 ISSN: 0027-8424
- M SALEH ET AL: "Generation of a human anti-idiotypic antibody that mimics the GD2 antigen" JOURNAL OF IMMUNOLOGY,US,THE WILLIAMS AND WILKINS CO. BALTIMORE, Bd. 151, Nr. 6, 15. September 1993 (1993-09-15), Seiten 3390-3398, XP002091381 ISSN: 0022-1767
- FAGERBERG J ET AL: "HUMAN ANTI-IDIOTYPIC ANTIBODIES INDUCED A HUMORAL AND CELLULAR IMMUNE RESPONSE AGAINST A COLORECTAL CARCINOMA-ASSOCIATED ANTIGEN IN PATIENTS" PROCEEDINGS OF THE NATIONAL ACADEMY OF SCIENCES OF USA,US,NATIONAL ACADEMY OF SCIENCE. WASHINGTON, Bd. 92, Nr. 11, 23. Mai 1995 (1995-05-23), Seiten 4773-4777, XP000566332 ISSN: 0027-8424
- Online catalogue of Amersham Biosciences: Product Hitrap(TM) Protein A HP columns, (24.03.2003).

## Beschreibung

Die vorliegende Erfindung beschreibt die Verwendung von Antikörpern, die an Antikörper gegen Tumor-assoziierte Antigene binden und die aus individuellen Antikörper enthaltenden Körperflüssigkeiten durch Immunaffinitätsreinigung an spezifischen Liganden gewonnen werden, zur Herstellung einer Zusammensetzung zur individuellen, autologen prophylaktischen oder therapeutischen Vakzinierung gegen Krebserkrankungen. Die Liganden der Immunaffinitätsreinigung sind Antikörper oder deren Derivate, die gegen ein oder mehrere Tumor-assoziierte Antigene gerichtet sind. Ferner betrifft die Erfindung pharmazeutische Zusammensetzungen, die die durch Immunaffinitätsreinigung erhaltenen Antikörper enthalten oder mit diesen in-vitro gepulste dendritische Zellen.

Das adaptive Immunsystem des Menschen besteht aus zwei wesentlichen Komponenten, der humoralen und der zellulären Immunität. Die adaptive Immunantwort beruht auf der clonalen Selektion von B- und T-Lymphozyten und erlaubt im Prinzip die Erkennung jedes beliebigen Antigens sowie den Aufbau eines immunologischen Gedächtnisses. Diese Merkmale des adaptiven Immunsystems werden generell bei Impfungen nutzbringend angesprochen.
Jede B-Zelle produziert einen Antikörper mit bestimmter Bindungsspezifität. Dieser Antikörper befindet sich als spezifischer Rezeptor auch in der Membran der ihn produzierenden B-Zelle. Die humorale Immunantwort gegen als fremd erkannte Antigene beruht auf der selektiven Aktivierung derjenigen B-Zellen, die solche Antikörper produzieren, die an Epitope des jeweiligen Antigens binden können. Für die Antikörpervielfalt spielen DNA-Rearrangements im Verlauf der B-Zelldifferenzierung eine entscheidende Rolle.
Im menschlichen Serum befinden sich in großer Menge Antikörper verschiedenster Spezifitäten, Isotypen und Subklassen. Die Gesamtkonzentration aller Immunglobuline im Serum beträgt 15 - 20 mg/ml; das heißt, daß ca. 100 g Immunglobuline verschiedenster Spezifitäten dauernd im Blut zirkulieren. Es ist nicht möglich, die genaue Anzahl aller Antikörper mit unterschiedlicher Spezifität anzugeben. Das theoretisch mögliche Repertoire dürfte bei ca. 10¹¹ liegen. Ein bestimmter Antikörper kann im allgemeinen verschiedene, einander ähnliche Antigene binden, wenn auch mit unterschiedlicher Affinität und Avidität.
Das Immunsystem muß mit Hilfe endogener Regulationsmechanismen eine Homöostase bezüglich der Verteilung und Gewichtung dieser verschiedenen Spezifitäten aufrecht erhalten. Ein wesentlicher Mechanismus dafür ist das "idiotypische Netzwerk", das von Niels Jeme vor ca. 25 Jahren postuliert wurde (Jerne, Ann. Immunol. 125C (1974), 373-389): Gegen jeden Idiotyp eines Antikörpers, welcher seine Bindungsspezifität bestimmt, gibt es anti-idiotypische Antikörper, die also an den Idiotyp des ersten Antikörpers im Sinne einer Antigenerkennung binden. Jeme schlug vor, daß die Wechselwirkungen zwischen den Idiotyp-spezifischen Rezeptoren auf Lymphozyten für die Regulation des Immunsystems verantwortlich sein können. Diese Wechselwirkungen finden offensichtlich tatsächlich statt, da gezeigt worden ist, daß im Zuge einer Immunantwort auch anti-idiotypische Antikörper gegen die durch die Immunantwort primär induzierten Antikörper entstehen. Da es gegen jeden Antikörper anti-idiotypische Antikörper gibt, sind Lymphozyten gegenüber Idiotypen von Antikörpern grundsätzlich nicht tolerant.
Einige anti-idiotypische Antikörper können im immunologischen Sinne das "innere Abbild" eines Antigens darstellen. Solche Antikörper können daher als Surrogat des nominalen Antigens zur Immunisierung verwendet werden, da die durch eine Immunisierung induzierten Antikörper zum Teil an das nominale Antigen binden können. Dieser Ansatz wird seit längerer Zeit insbesondere in der Krebsimmuntherapie verfolgt. Es gibt seit mehr als 10 Jahren eine Reihe von präklinischen und klinischen Projekten, durch Vakzinierung mit monoclonalen anti-idiotypischen Antikörpern Immunantworten gegen Tumor-assoziierte Antigene hervorzurufen (für eine Zusammenfassung siehe: Bhattacharya-Chatterjee, Foon; Anti-idiotype antibody vaccine therapies of cancer; Cancer Treat. Res. 94 (1998), 51-68). Die meisten dieser monoclonalen anti-idiotypischen Antikörper sind murinen Ursprungs, es gibt aber auch Versuche mit humanen monoclonalen anti-idiotypischen Antikörpern (z.B. Fagerberg et al., PNAS 92 (1995), 4773-4777).
Einen Spezialfall stellen die Versuche der therapeutischen Immunisierung von Patienten mit B-Zell Lymphomen dar. Bei diesen Erkrankungen entartet ein bestimmter B-Zell Clon, der ein bestimmtes Immunglobulin mit einem dafür charakteristischen Idiotyp produziert und auch als Rezeptor in der Zellmembran trägt. Dieser Antikörper ist monoclonal und kann als Tumor-spezifisches Antigen für das individuelle B-Zell Lymphom gesehen werden. Es wurde gezeigt, daß solche Patienten-spezifische autologe Antikörper, in Zellkultur hergestellt und in geeigneter immunogener Form als Impfstoff verabreicht, eine Immunantwort gegen den Idiotyp dieses Antikörpers hervorrufen können, die eine Wirkung gegen das B-Zell Lymphom aufweisen kann (Reichardt et al., Blood. 93 (1999), 2411-2419).
Zusammenfassend kann gesagt werden, daß
- humane monoclonale anti-idiotypische Antikörper, die ein Tumor-assoziiertes Antigen nachahmen, als Impfstoff in Krebspatienten eine Immunantwort auslösen können, die gegen dieses Tumor- assoziierte Antigen gerichtet sein kann. Solche humane monoclonale Antikörper wurden mittels Hybridomtechnologie oder Immortalisierung humaner B-Zellen aus Patienten gewonnen, die einen (meist murinen) Antitumor-Antikörper zur passiven Immuntherapie erhalten hatten und eine immunantwort dagegen aufgebaut haben. Mit so hergestellten humanen monoclonalen anti-idiotypischen Antikörpern wurden dann andere Patienten immunisiert.
- humane Antikörper, die von einem B-Zell Lymphom produziert werden und daher einen definierten idiotyp haben, Patienten-spezifisch als individuelle Impfung eine immunantwort auslösen können, die gegen das B-Zell Lymphom gerichtet sein kann. Solche Patienten-spezifische Antikörper müssen mittels Hybridomtechnologie oder Immortalisierung der Zellen des B-Zell Lymphoms individuell gewonnen werden. Ihre Verwendung ist naturgemäß auf die Behandlung von B-Zell Lymphomen beschränkt.

Es ist somit zwar bekannt, daß monoclonale anti-idiotypische, Antikörper aus einer bestimmten Spezies zur Immunisierung auch von Individuen der gleichen Spezies, aus der die Antikörper stammen, verwendet werden können. Hierzu müssen aber in jedem Fall Zellen manipuliert und kultiviert werden. Somit ist dieser Ansatz aufwendig und auf monoclonale Antikörper beschränkt.

In Losman et al. Proc., Natl. Acad. Sci. USA 88 (1991), 3421-3425, wird die Reinigung von Ab2-Antikörpern aus einem Krebspatienten beschrieben. Dieser Patient war allerdings zuvor mit einem murinen monoclonalen Antikörper (NP-4) gegen CEA behandelt worden.

Es ist somit klar, daß nach wie vor ein Bedarf besteht, Methoden und Mittel zur Verfügung zu stellen, die eine effektive und breit anwendbare und gegebenenfalls. individuelle Behandlung von Tumoren sowie einen Schutz vor Krebserkrankungen ermöglichen.

Der vorliegenden Erfindung liegt somit die Aufgabe zugrunde, effiziente und im Rahmen verschiedener Tumorarten individuell einsetzbare Mittel und Verfahren zur Behandlung von Krebs sowie zum Schutz vor Krebserkrankungen bereitzustellen.

Diese Aufgabe wird durch die Bereitstellung der in den Ansprüchen gekennzeichneten Ausführungsformen gelöst.

Somit betrifft die vorliegende Erfindung die Verwendung von Antikörpern zur Herstellung einer Zusammensetzung, die geeignet ist als Impfstoff zur therapeutischen oder prophylaktischen Impfung gegen Krebs, dadurch gekennzeichnet, daß die Antikörper aus Antikörper enthaltenden Körperflüssigkeiten durch Immunaffinitätsreinigung gewonnen werden, wobei als Liganden für die Immunaffinitätsreinigung Antikörper verwendet werden, die ein oder mehrere Tumor-assoziierte Antigene erkennen, oder deren Fragmente mit gleichem Idiotyp, , und wobei die Körperflüssigkeit aus einem Individuum stammt, das nicht zuvor einen monoclonalen Antikörper verabreicht bekommen hat, der gegen eines der Tumor-assoziierten Antigene gerichtet ist.

Die erfindungsgemäße Verwendung hat gegenüber den im Stand der Technik beschriebenen Methoden den Vorteil, daß zur Herstellung der anti-idiotypischen Antikörper keine Zellkulturen verwendet werden. Dadurch erlaubt das erfindungsgemäße Konzept eine breite und effektive Anwendung und ist nicht auf monoclonale Antikörper beschränkt.
Der Pool von Antikörpern, welche z.B. in insgesamt großer Menge im Blut jedes Menschen vorhanden sind, beinhaltet Antikörper jeder möglichen Bindungsspezifität. Daher beinhaltet dieser Antikörperpool grundsätzlich auch Antikörper (Ab2) gegen den Idiotyp von (bereits bekannten oder aber auch neuen) z.B. murinen monoclonalen Antikörpern (Ab1) gegen beliebige Tumor-assoziierte Antigene (TAA). Entsprechend dem immunologischen Netzwerk enthält der gleiche Antikörperpool in jedem Menschen in gewisser Menge auch Antikörper gegen die Idiotypen der autologen Ab2, die sogenannten Ab3-Antikörper. Ab3 können nach der Netzwerktheorie an dem Tumor-assoziierten Antigen binden, das durch den externen Ab1 definiert ist. Eine Verschiebung des Gleichgewichtes Ab2 / Ab3 zugunsten von Ab3 muß zur Folge haben, daß das Immunsystem Tumorzellen, die dieses Antigen tragen, besser erkennen und angreifen kann.
Basis der vorliegenden Erfindung ist nun, daß eine solche Verschiebung des immunologischen Gleichgewichtes durch Verabreichung einer individuellen autologen Ab2-Fraktion in Form einer immunogenen Vakzine erreicht werden kann. Durch eine solche autologe Impfung können diejenigen B-Zellen selektiv stimuliert werden, die Ab3 produzieren. Für eine solche Impfung sind nur kleine Mengen der Ab2-Fraktion notwendig, die nach an sich bekannten Methoden mit einem geeigneten Vakzinadjuvans immunogen formuliert werden können.
Bei den erfindungsgemäß verwendeten Antikörpern handelt es sich somit um Ab2-Antikörper aus Antikörper enthaltenden Körperflüssigkeiten, die gegen den zur Immunaffinitätsreinigung eingesetzten anti-TAA-Antikörper gerichtet sind. Körperflüssigkeiten, die Antikörper enthalten, sind beispielsweise Blut, Plasma, Serum, Lymphflüssigkeit, maligne Ergüsse etc. Bevorzugt ist die Körperflüssigkeit Serum. Die Körperflüssigkeit kann dabei aus jedem tierischen Organismus stammen, der Körperflüssigkeiten besitzt, die Antikörper enthalten. Bevorzugt sind dies Vertebraten, besonders bevorzugt Säugetiere und ganz besonders bevorzugt ist die Körperflüssigkeit menschlichen Ursprungs.
Da die durch Vakzinierung mit autologen Antikörpern induzierte Immunantwort durch die Bindungsregion dieser Antikörper, d.h. durch ihren Idiotyp, determiniert wird, können im Prinzip statt intakter Antikörperfraktionen auch Fragmente oder Derivate dieser Antikörper erfolgreich zur Vakzinierung eingesetzt werden, solange diese den Idiotyp der jeweiligen Ausgangs-Antikörper weiterhin beinhalten. Der Begriff "Antikörper" umfaßt daher auch Fragmente oder Derivate solcher Antikörper mit der gleichen Bindungsspezifität. Als Beispiele, jedoch nicht auf diese eingeschränkt, seien erwähnt: F(ab)₂'-Fragmente und F(ab)'-Fragmente, die z.B. nach an sich bekannten biochemischen Methoden (beispielsweise durch enzymatische Spaltung) hergestellt werden können. Der Begriff "Derivat" umfaßt dabei z.B. Antikörperderivate, die nach an sich bekannten chemischen oder biochemischen Methoden hergestellt werden können, wie z.B. mit Fettsäuren an freien Aminofunktionen amidierte Antikörper zwecks Erhöhung der Lipophilie zur Inkorporierung in Liposomen. Insbesondere umfaßt der Begriff auch Produkte, die erzeugt werden können durch chemische Kopplung von Antikörpern oder Antikörperfragmenten mit Molekülen, die die Immunantwort verstärken können, wie z.B. Tetanus-Toxoid, Pseudomonas Exotoxin, Derivate von Lipid A, GM-CSF, IL-2 oder durch chemische Kopplung von Antikörpern oder Antikörperfragmenten mit Polypeptiden, die die Immunantwort verstärken, wie z.B. GM-CSF, IL-2, IL-12, C3d etc. Die Reinigung der Antikörper aus Körperflüssigkeiten, die Antikörper enthalten, z.B. aus menschlichem Serum, mittels Immunaffinitätsreinigung kann dabei nach dem Fachmann bekannten Methoden erfolgen (Clin. Chem. 45 (1999), 593; J. Chem. Technol. Biotechnol. 48 (1990), 105). Dabei wird ein TAA-spezifischer Antikörper, bzw. ein Gemisch von Antikörpern, an einer Festphase immobilisiert. Die Festphase kann dabei eine Membrane, ein Gel oder ähnliches Material sein, an das Antikörper ohne wesentlichen Verlust der Bindungseigenschaften gebunden werden können. Die Bindung der Ab2 an die immobilisierten Antikörper kann batchweise oder im Durchfluß-Verfahren erfolgen. Die Immunaffinitätsreinigung kann an einer Chromatographie-Apparatur automatisch oder mittels eines manuellen Verfahrens erfolgen. Es ist aber auch denkbar, daß das Verfahren mittels einer einfachen Vorrichtung, die die immobilisierten Antikörper enthält, manuell, automatisch oder halbautomatisch durchgeführt wird. Bei einer Immunaffinitätschromatographie, in Beispiel 1 beschrieben, erhält man in der Regel zwischen 3 - 10 µg Immunglobulin pro ml eingesetzten Serums. Im allgemeinen besteht die so erhaltene Antikörperfraktion sowohl aus IgM als auch aus IgG. Es ist also möglich, aus einer für eine Blutabnahme akzeptablen Menge von z.B. 50 ml, die zu ca. 25 ml Serum führt, ca. 0,08 - 0,25 mg Ab2 zu gewinnen. Diese Menge reicht an sich bereits zur Vakzinierung. Falls es gewünscht ist, größere Mengen von Ab2 zu gewinnen, können auch an sich bekannte Techniken der Plasmapherese, kombiniert mit Immunaffinitätsreinigung, eingesetzt werden. Wenn mehrere Antikörper verschiedener Spezifität verwendet werden, können diese immobilisierten Antikörper simultan verwendet werden oder einzelne Immunaffinitätsreinigungen parallel oder seriell erfolgen.
Unter dem Begriff "Tumor-assoziiertes Antigen" wird im Rahmen der vorliegenden Anmeldung eine Struktur verstanden, die bevorzugt von Tumorzellen präsentiert wird und dadurch eine Unterscheidung zu nicht-malignem Gewebe ermöglicht. Vorzugsweise ist ein solches Tumor-assoziiertes Antigen auf bzw. in der Zellmembran einer Tumorzelle lokalisiert. Dabei ist aber nicht ausgeschlossen, daß derartige Antigene auch auf nicht-entarteten Zellen vorkommen. Bei Tumor-assoziierten Antigenen kann es sich beispielsweise um Polypeptide, insbesondere glykosylierte Proteine, oder Glykosylierungsmuster von Polypeptiden handeln. Andere Strukturen, die ein Tumor-assoziiertes Antigen darstellen können, sind z.B. Glykolipide. Hierzu gehören beispielsweise Ganglioside, wie z.B. GM2. Ferner können Tumor-assoziierte Antigene dargestellt werden durch Veränderungen der Zusammensetzung von Lipiden der Zellmembran, die für Krebszellen charakteristisch sein können.
Verschiedenste Antikörper gegen verschiedene Tumor-assoziierte Antigene sind seit langem bekannt oder können mit an sich bekannten Methoden (z.B. Hybridomtechnologie, Phagendisplay-Technik) hergestellt werden.

In einer bevorzugten Ausführungsform werden die durch Immunaffinitätsreinigung erhaltenen Antikörper im Sinne eines autologen, individuellen Impfstoffes jeweils demjenigen Individuum verabreicht, aus deren Körperflüssigkeit sie gewonnen worden sind.
Ein weiterer integraler Bestandteil der vorliegenden Erfindung ist somit, daß die Gewinnung einer Ab2-Fraktion aus der Körperflüssigkeit, z.B. Blut, eines Individuums durch Immunaffinitätsreinigung dieser in der Körperflüssigkeit befindlichen Antikörper erfolgen kann. Als Ligand wird ein - oder werden mehrere -Antikörper gegen Tumor-assoziierte Antigene verwendet.
Ein Individuum kann in diesem Zusammenhang jeder beliebige tierische Organismus sein, vorzugsweise ein Vertebrat, besonders bevorzugt ein Säuger und ganz besonders bevorzugt ein Mensch, und wobei, das Individuum nicht zuvor einen monoclonalen Antikörper verabreicht bekommen hat, der gegen eines der Tumor-assoziierten Antigene gerichtet ist.

In einer weiteren bevorzugten Ausführungsform werden für die Immunaffinitätsreinigung gleichzeitig mehrere Antikörper gegen verschiedene Tumor-assoziierte Antigene und/oder gegen verschiedene Epitope eines oder mehrerer Tumor-assoziierter Antigene verwendet.
Werden für die Immunaffinitätsreinigung der in Körperflüssigkeiten befindlichen Ab2 mehrere, insbesondere monoclonale, Antikörper gegen verschiedene Tumor-assoziierte Antigene oder gegen verschiedene Epitope gleichzeitig verwendet, so erhält man eine Fraktion unterschiedlicher Ab2, die auf dem gewählten Set von Tumor-assoziierten Antigenen beruht und deren gleichzeitige Verwendung als Impfstoff eine Immunantwort gegen alle diese Tumor-assoziierten Antigene oder Epitope hervorruft. Damit wird also das immunologische Gleichgewicht in einem Schritt in Richtung der Erkennung eines Sets von (oft co-exprimierten) Tumor-assoziierten Antigenen oder Epitopen verschoben. Eine solche Vorgangsweise erhöht naturgemäß die Wirksamkeit der induzierten Immunantwort und verringert essentiell die Ausbildung von Antigen-negativen Varianten des Tumors ("tumor escape"), da es extrem unwahrscheinlich ist, daß eine Tumorzelle die Expression mehrerer Antigene gleichzeitig abschalten kann.
Grundsätzlich kann die oben beschriebene Methode auf allen bekannten oder neu entdeckten Tumor-assoziierten Antigenen beruhen. Voraussetzung ist nur, daß ein oder mehrere Antikörper, vorzugsweise monoclonale Antikörper, gegen ein solches Antigen verfügbar sind, die als Ligand für eine Immunaffinitätsreinigung verwendet werden können. Es eignen sich grundsätzlich alle denkbaren Arten von Antikörpern, insbesondere polyclonale oder monoclonale, wobei monoclonale Antikörper bevorzugt sind. Es könne auch Mischungen von polyclonalen und monoclonalen Antikörpern eingesetzt werden. Auch können sowohl z.B. murine monoclonale Antikörper als auch Antikörper aus anderen Spezies (z.B. Ratte) verwendet werden. Weiter sind auch Maus-Human chimärische, humanisierte oder humane monoclonale Antikörper gegen Tumor-assoziierte Antigene einsetzbar. Die Eigenschaft der für die Immunaffinitätsreinigung eingesetzten Antikörper ist durch ihre Bindungsregion, das heißt ihrem Idiotyp, determiniert. Daher können im Prinzip statt intakter Antikörper auch Fragmente dieser Antikörper erfolgreich zur Immunaffinitätsreinigung eingesetzt werden, solange diese Fragmente den Idiotyp der jeweiligen Ausgangs-Antikörper weiterhin beinhalten. Als Beispiele, jedoch nicht auf diese eingeschränkt, seien erwähnt:, F(ab)'₂-Fragmente, F(ab)'-Fragmente und Fv-Fragmente, die nach an sich bekannten biochemischen Methoden (enzymatische Spaltung) oder nach an sich bekannten molekularbiologischen Methoden hergestellt werden können. Es können selbstverständlich Mischungen der jeweiligen Arten von Antikörpern oder Fragmente eingesetzt werden.
Die Auswahl der Tumor-assoziierten Antigene und damit der für die Immunaffinitätsreinigung eingesetzten Antikörper gegen diese Antigene hängt mit der Antigencharakteristik der Tumorindikation zusammen, gegen die geimpft werden soll.

Folgende bereits bekannte Tumor-assoziierte Antigene, die auf verschiedenen, insbesondere epithelialen Tumoren häufig exprimiert sind, sind beispielhaft angeführt, aber die Anwendung der hier beschriebenen Methode der autologen Vakzinierung ist in keiner Weise auf diese Antigene beschränkt:
Epitheliales Zell Adhäsions Molekül (Ep-CAM)
Carcino Embryonales Antigen (CEA)
Lewis Y Kohlenhydrat
Sialyl Tn Kohlenhydrat
Globo H Kohlenhydrat
Ganglioside wie GD2 / GD3 / GM2
Prostate Specific Antigen (PSA)
CA 125
CA 19-9
CA 15-3
TAG-72
EGF-Rezeptor
Neuronales Zell Adhäsions Molekül (N-CAM)
Her2/Neu Rezeptor
D 97
CD 20
CD 21

Gegen alle genannten Antigene sind (meistens mehrere), insbesondere monoclonale, Antikörper beschrieben worden, die sich im Prinzip alle als Liganden für die oben beschriebene Immunaffinitätsreinigung zur Gewinnung von Ab2 eignen. Weitere Tumor-assoziierte Antigene werden z.B. beschrieben in DeVita et al. (Eds., "Biological Therapy of Cancer", 2. Auflage, Kapitel 3: Biology of Tumor Antigenes, Lippincott Company, ISBN 0-397-5144116-6 (1995)).

In einer bevorzugten Ausführungsform ist das Tumor-assoziierte Antigen ein membranständiges Molekül, das auf Krebszellen epithelialen Ursprungs, auf Krebszellen neuro-endokrinen Ursprungs oder auf Krebszellen des blutbildenden Systems häufig exprimiert oder co-exprimiert wird.

In einer weiteren bevorzugten Ausführungsform ist die erfindungsgemäß hergestellte Zusammensetzung geeignet für eine mehrfache Verabreichung der darin enthaltenen Antikörper.
Die hier beschriebene Methode zur Gewinnung eines autologen Impfstoffes aus Körperflüssigkeiten eines Individuums ist naturgemäß nicht auf nur eine Anwendung beschränkt.
Die durch eine erste Impfung hervorgerufene Verschiebung des immunologischen Repertoires in Richtung Antitumor-Wirksamkeit kann durch Wiederholungen dieses Vorganges weiter verstärkt werden, z.B. kann einige Wochen nach Gewinnung der ersten autologen Vakzine durch Immunaffinitätsreinigung erneut Körperflüssigkeit, z.B. Blut, abgenommen und erneut eine autologe Vakzine präpariert und verabreicht werden. Dadurch wird auch gewährleistet, daß der jeweilige Status des immunologischen Gleichgewichtes in der individuellen Vakzine immer berücksichtigt ist. Dieser Vorgang kann in geeigneten Abständen immer wieder durchgeführt werden (beispielhaft zunächst alle 4 - 8 Wochen, in weiterer Folge alle 6 Monate).

Die hier beschriebene neue Zusammensetzung und Methode zur Impfung mit autologen Antikörpern eignet sich grundsätzlich sowohl für therapeutische als auch prophylaktische Zwecke. Eine wiederholte therapeutische Vakzinierung von Krebspatienten kann die Ausbildung neuer Metastasen unterdrücken und die Disseminierung der Erkrankung zumindestens verlangsamen. Therapeutische autologe Impfungen können insbesondere in folgenden Stadien der Erkrankung nutzbringend sein:
In frühen Krankheitsstadien, zum Beispiel nach erfolgreicher Operation eines Primärtumors (adjuvantes Stadium), werden durch die hier beschriebenen autologen Impfungen restliche, disseminierte Tumorzellen zerstört und daran gehindert, sich als neue Metastasen zu etablieren. Dadurch kann die rückfallsfreie Lebensspanne und damit auch die Gesamtüberlebenszeit solcher Patienten verlängert werden. Durch solche autologe Impfungen und in geeigneten Abständen durchgeführte Auffrischungsimpfungen kann gegebenenfalls ein lebenslanger Schutz vor der Ausbildung von Metastasen erreicht werden.
Wenn bereits Metastasen aufgetreten sind, kann durch die hier beschriebene Zusammensetzung bzw. die autologen Impfungen die Ausbreitung und Ausbildung weiterer Metastasen eingedämmt werden. Dadurch wird das Krankheitsgeschehen stabilisiert, wobei bei Erhalt der Lebensqualität eine Verlängerung der Lebenszeit erreicht werden kann.

Die intrinsische Induktion von Ab3 mit potentiellen Antitumoreigenschaften via Induktion von Ab2 durch passive immuntherapie mit murinen Antikörpern gegen Tumor-assoziierte Antigene (Ab1), die i.v. an Krebspatienten gegeben werden, wird seit vielen Jahren postuliert und untersucht (z.B. Koprowski et al., PNAS 81 (1984), 216-19).
Die hier beschriebene Zusammensetzungund die autologen impfungen können prinzipiell auch dafür verwendet werden, prophylaktisch einen erhöhten Schutz gegen die Ausbildung von Krebserkrankungen in gesunden Individuen aufzubauen. Eine solche Maßnahme kann sich insbesondere in Risikogruppen als sinnvoll erweisen (hierzu gehören z.B. Individuen mit einer genetischen Disposition, bestimmte Krebserkrankungen zu entwickeln, was in steigendem Ausmaß durch entsprechende Tests erhoben werden kann).
Ein genereller Vorzug der hier beschriebenen Strategie der individuellen autologen Impfung liegt in der Tatsache, daß der immunologische Status des jeweiligen Individuums bezüglich des idiotypischen Netzwerkes berücksichtigt wird, da der entsprechende Impfstoff vorzugsweise jeweils aus der individuellen Körperflüssigkeit, z.B. Serum, hergestellt wird.

Desweiteren kommt das immunisierte Individuum dabei mit keinen Fremdantigenen in Berührung, sondern wird in geeigneter Form nur mit körpereigenen Bestandteilen behandelt, die eine Modulation des immunologischen Gleichgewichtes bewirken.

In einer bevorzugten Ausführungsform wird erfindungsgemäß der durch Immunaffinitätsreinigung erhaltene Antikörper mit einem geeigneten Vakzineadjuvans formuliert.
Wie bei Impfstoffen üblich, können die autologen Antikörperfraktionen oder deren Fragmente und Derivate mit Vakzine-Adjuvantien gemeinsam formuliert werden. Durch solche Adjuvantien wird die Immunantwort verstärkt. Als Beispiele für Adjuvantien, jedoch nicht auf diese eingeschränkt, seien erwähnt: Aluminium-haltige Adjuvantien, insbesondere Aluminium-hydroxid (z.B. Alu-Gel), Derivate von Lipopolysaccharid, Bacillus Calmette Guerin (BCG), QS-21, Liposomenbereitungen, Formulierungen mit zusätzlichen Antigenen, gegen die das Immunsystem bereits eine starke Immunantwort gemacht hat, wie z.B. Tetanus Toxoid oder Bestandteile von Influenza Viren, gegebenenfalls in einer Liposomenbereitung.
Die Impfstoffbereitung kann zur Verstärkung der Immunantwort auch mit entsprechenden, vorzugsweise humanen, Zytokinen, die den Aufbau einer Immunantwort unterstützen, verabreicht werden. Hier ist insbesondere, wenn auch nicht ausschließlich, Granulozyten-Makrophagen stimulierender Faktor (GM-CSF) zu erwähnen. Dieses Zytokin stimuliert durch Aktivierung von Antigen-prozessierenden Zellen (z.B. dendritische Zellen) eine effiziente Immunantwort.
Gegebenenfalls können die autologen Antikörperfraktionen auch nach an sich bekannten und publizierten Methoden mit autologen, ex-vivo kultivierten dendritischen Zellen inkubiert werden. Die so gepulsten dendritischen Zellen werden anschließend dem jeweiligen Individuum wieder verabreicht. Auf diese Weise kann eine besonders effiziente Immunantwort erreicht werden.

In einer bevorzugten Ausführungsform der erfindungsgemäßen Verwendung werden die in der Zusammensetzung enthaltenen Antikörper mit einem Adjuvans gemischt und anschließend einer Hitzebehandlung vorzugsweise bei einer Temperatur zwischen 70 und 121°C unterzogen. Das verwendete Adjuvans ist vorzugsweise ein aluminiumhaltiges Adjuvans. Es ist möglich, daß eine derartige Hitzebehandlung das Protein-Antigen zwar denaturiert, die immunogenen Teile des Proteins aber durch die Bindung an das Adjuvans dem Immunsystem in der richtigen Form präsentiert werden können. Es ist aber nicht unbedingt notwendig, die Proteine zu denaturieren um die Vorteile einer Hitzebehandlung zu erhalten. Es ist bekannt, daß die thermische Denaturierung von Proteinen nicht nur von der Temperatur, sondern auch von der Zeit, in der das Protein dieser Temperatur ausgesetzt ist, abhängt. Darüberhinaus sind noch weitere physikalisch chemische Parameter, wie z.B. Ionenstärke, Ionenzusammensetzung, pH-Wert, Art und Menge der aktiven Oberfläche im Gemisch für die Denaturierung eines Proteins verantwortlich. Es sind Bedingungen vorstellbar, bei denen die Antikörper nicht oder nicht vollständig denaturiert werden und/oder andere Effekte, wie zum Beispiel schwächere Desorption von der Oberfläche des Adjuvans, genützt werden können.
Ein weiterer Vorteil einer derartigen Herstellungsweise einer Impfstoffformulierung mit einem Adjuvans und der darauffolgenden Hitzebehandlung ist, daß in der gesamten Formulierung infektiöse Erreger abgeschwächt, bzw. inaktiviert werden könnten. Dieser Vorteil kann sowohl in der Herstellung als auch bei der Lagerung und Distribution der Impfstoffformulierung eine Rolle spielen. Es ist damit eine größere Sicherheit in Bezug auf bekannte und unbekannte Erreger von übertragbaren Krankheiten gegeben. Darüberhinaus ist, bei entsprechender Verpackung eine Abfüllung ohne Konservierungsstoffe möglich, da die mikrobielle Konservierung des Impfstoffes durch Hitze erfolgt ist.
Ein weiterer Vorteil einer derartigen Formulierung ist die mögliche erhöhte Immunogenität der Antikörper, da die Erhitzung eine zumindest teilweise Denaturierung der Antikörper bewirken kann. Diese erhöhte Antigenität kann im Besonderen bei Proteinen, die vom Immunsystem als eigene Proteine erkannt werden würden, die Immunogenität erhöhen.
Ein weiterer Vorteil ist eine mögliche zusätzliche Stabilisierung des Antikörper-Adjuvans Komplexes durch die Hitzeinaktivierung, d.h. die Desorption des Protein-Antigens erfolgt nicht mehr so schnelt wie in Antigen-Adjuvans Formulierungen, die nicht hitzebehandelt worden sind. Dieser Vorteil erlaubt unter Umständen auch einen größeren zeitlichen Abstand zwischen den einzelnen Immunisierungen.

Die erfindungsgemäß hergestellte Zusammensetzung kann nach gängigen Methoden verabreicht werden, z.B. als Impfstoff durch subkutane oder intramuskuläre Injektion.

Die vorliegende Erfindung betrifft ebenfalls pharmazeutische Zusammensetzungen enthaltend IgM und IgG Antikörper, die aus Antikörper enthaltenden Körperflüssigkeiten durch Immunaffinitätsreinigung gewonnen wurden, wobei als Liganden für die Immunaffinitätsreinigung Antikörper verwendet werden, die ein oder mehrere Tumorassoziierte Antigene erkennen, oder deren Fragmente mit gleichem idiotyp und wobei die Körperflüssigkeit aus einem Individuum stammt, das nicht zuvor einen monoclonalen Antikörper verabreicht bekommen hat, der gegen eines der Tumor-assoziierten Antigene gerichtet ist.

Derartige Zusammensetzungen eignen sich wie oben beschrieben als Impfstoffe zur therapeutischen oder prophylaktischen Impfung gegen Krebserkrankungen.
Für die bevorzugten Ausführungsformen der Herstellung, der Bestandteile, der Formulierung, der Verabreichungsarten etc. der pharmazeutischen Zusammensetzung gilt das, was oben bereits im Zusammenhang mit der erfindungsgemäßen Verwendung ausgeführt wurde.

Schließlich betrifft die Vorliegende Erfindung auch eine pharmazeutische Zusammensetzung enthaltend isolierte, ex-vivo kultivierte dendritische Zellen eines Individuums, wobei die dendritischen Zellen zuvor mit Antikörpern in-vitro inkubiert wurden, welche wie oben beschrieben aus einer Antikörper enthaltenden Körperflüssigkeit desselben Individuums durch Immunaffinitätsreinigung gewonnen wurden.

Die obenbeschriebenen Antikörperpräparationen können erfindungsgemäß eingesetzt werden in einem Verfahren zur therapeutischen oder prophylaktischen Impfung gegen Krebs, wobei einem Individuum Antikörper verabreicht werden, die aus Antikörper enthaltenden Körperflüssigkeiten, vorzugsweise aus Körperflüssigkeiten desselben Individuums, durch Immunaffinitätsreinigung gewonnen wurden, wobei als Liganden für die Immunaffinitätsreinigung Antikörper verwendet werden, die ein oder mehrere Tumor-assoziierte Antigene erkennen, oder deren Fragmente mit gleichem Idiotyp , und wobei die Körperflüssigkeit aus einem Individuum stammt, das nicht zuvor einen monoclonalen Antikörper verabreicht bekommen hat, der gegen eines der Tumor-assoziierten Antigene gerichtet ist.

Die obenbeschriebenen dendritischen Zellen können erfindungsgemäß eingesetzt werden in einem Verfahren zur therapeutischen oder prophylaktischen Impfung gegen Krebs, wobei einem Individuum autologe ex-vivo kultivierte, isolierte dendritische Zellen verabreicht werden, die zuvor in-vitro mit Antikörpern inkubiert wurden, welche aus Antikörper enthaltenden Körperflüssigkeiten durch Immunaffinitätsreinigung gewonnen wurden, wobei als Liganden für die Immunaffinitätsreinigung Antikörper verwendet werden, die ein oder mehrere Tumor-assoziierte Antigene erkennen, oder deren Fragmente mit gleichem idiotyp , und wobei die Körperflüssigkeit aus einem Individuum stammt, das nicht zuvor einen monoclonalen Antikörper verabreicht bekommen hat, der gegen eines der Tumor-assoziierten Antigene gerichtet ist.

Für die bevorzugten Ausführungsformen gilt das bereits oben im Zusammenhang mit der erfindungsgemäßen Verwendung Gesagte.

Die vorliegende Erfindung betrifft auch ein Verfahren zur Herstellung einer Antikörperpräparation dadurch gekennzeichnet, daß aus einer Antikörper enthaltenden Körperflüssigkeit durch Immunaffinitätsreinigung Antikörper gewonnen werden, wobei als Liganden für die Immunaffinitätsreinigung Antikörper verwendet werden, die ein oder mehrere Tumor-assoziierte Antigene erkennen, oder deren Fragment mit gleichem idiotyp, , und wobei die Körperflüssigkeit aus einem Individuum stammt, das nicht zuvor einen monoclonalen Antikörper verabreicht bekommen hat, der gegen eines der Tumor-assoziierten Antigene gerichtet ist.

Für die Körperflüssigkeiten, die als Liganden verwendeten Antikörper sowie für die Tumor-assoziierten Antigene gilt dasselbe, was bereits oben im Zusammenhang mit der erfindungsgemäßen Verwendung gesagt wurde.
Die Immunaffinitätsreinigung kann nach dem Fachmann bekannten Methoden durchgeführt werden. Auch hier gilt das bereits im Zusammenhang mit der erfindungsgemäßen Verwendung gesagte.

In einer bevorzugten Ausführungsform des erfindungsgemäßen Verfahrens werden für die Affinitätsreinigung gleichzeitig mehrere Antikörper gegen verschiedene Tumor-assoziierte Antigene und/oder Epitope solcher Antigene verwendet. Hierbei können bei dem Ansatz, bei dem mehrere Antikörper für die Affinitätsreinigung verwendet werden, diese entweder simultan eingesetzt werden oder einzelne Immunaffinitätsreinigungen parallel oder seriell erfolgen. D.h. es können verschiedene Epitop- und/oder Antigen-Spezifitäten den Anforderungen entsprechend kombiniert werden. Diese Anforderungen ergeben sich aus der Expression verschiedener Tumorantigene auf verschiedenen individuellen Tumoren.

In einer besonders bevorzugten Ausführungsform werden als Liganden in der Affinitätsreinigung monoclonale Antikörper verwendet und bei einer ganz besonders bevorzugten Ausführungsform wird als Körperflüssigkeit Serum verwendet.

Bei einem erfindungsgemäßen Verfahren handelt es sich vorzugsweise um ein Verfahren zur Herstellung einer Antikörperpräparation, die verwendet wird für die Herstellung einer pharmazeutischen Zusammensetzung geeignet als Impfstoff zur therapeutischen oder prophylaktischen impfung gegen Krebs.

Ferner betrifft die vorliegende Erfindung ein Verfahren zur Herstellung einer pharmazeutischen Zusammensetzung, wobei ein oben beschriebenes erfindungsgemäßes Verfahren zur Herstellung einer Antikörperpräparation durchgeführt wird und die so erhaltene Antikörperpräparation anschließend mit einem pharmazeutisch verträglichen Träger und/oder einem geeigneten Vakzineadjuvans formuliert wird.

In einer bevorzugten Ausführungsform wird die Antikörperpräparation mit einem Adjuvans, vorzugsweise einem aluminiumhaltigen Adjuvans, gemischt und anschließend einer Hitzebehandlung, vorzugsweise bei einer Temperatur zwischen 70 und 121°C, unterworfen.

Für bevorzugte Ausführungsformen einer solchen pharmazeutischen oder Impfstoffformulierung gilt das oben im Zusammenhang mit der erfindungsgemäßen Verwendung Gesagte.

Der Offenbarungsgehalt aller in der vorliegenden Anmeldung zitierten Patente, Publikationen oder Datenbankeinträge ist hiermit unter Bezugnahme in seiner Gesamtheit in den Offenbarungsgehalt der vorliegenden Anmeldung eingeschlossen.
- **Figur 1**: zeigt ein Chromatogramm einer Immunaffinitätsreinigung (mittels immobilisiertem Antikörper 17-1A) der Antikörperfraktion aus Serum eines Rhesusaffen.
- **Figur 2**: zeigt ein Chromatogramm (Größentrennungschromatographie) einer Immunaffinitätsgereinigten Antikörperfraktion.
- **Figur 3**: zeigt die Bindung von Serum-Antikörpern, die durch Affinitätsreinigung an Antikörper 17-1A gewonnen wurden: 17-1A ELISA.
- **Figur 4**: zeigt das Ergebnis einer autologen Impfung eines Rhesusaffen: Bindung von Serum-Ig an KatoIII Tumorzellen (Zell-ELISA).
- **Figur 5**: zeigt ein Chromatogramm (Größentrennungschromatographie) einer immunaffinitätsgereinigten Antikörperfraktion (Mischbett anti-EpCAM/anti-Lewis Y).
- **Figur 6**: zeigt ein Chromatogramm eines Größenstandards (aufgetrennt durch Größentrennungschromatographie).

In den nun folgenden Beispielen, die die vorliegende Erfindung weiter erläutern, aber nicht einschränken sollen, wurden folgende Materialien verwendet:
- Mikrotiterplatten:: Immuno Plate F96 MaxiSorp (Nunc) für ELISA Cell Culture Cluster (Costar; Cat.Nr. 3598) für Zell-ELISA
- Zellinie:: KATO III: menschliche Magenkrebszellinie
(ATCC HTB 103)
- Kopplungspuffer:: 0,1 M NaHCO₃
0,5 M NaCl
pH-Wert 8,0
- Reinigungspuffer A:: PBS def
0,2 M NaCl
- Reinigungspuffer B:: 0,1 M Glycin / HCl
0,2 M NaCl
pH-Wert 2,9
- Medium A:: RPMI 1640 + 2 g/l NaHCO₃
100 U/ml Penicillin G
100 µg/ml Streptomycinsulfat
4 mM Glutamin
10 % foetales Kälberserum (hitzeinaktiviert)
- Bindungspuffer:: 15 mM Na₂CO₃
35 mM NaHCO₃
3 mM NaN₃
pH-Wert: 9,6
- PBS deficient (def):: 138 mM NaCl
1,5 mM KH₂PO₄
2,7 mM KCl
6,5 mM Na₂HPO₄
pH-Wert: 7,2
- Fixierlösung:: 0,1 % Glutardialdehyd in
physiologischer NaCl-Lösung
- Waschpuffer A:: 2% NaCl
0,2% Triton X-100
in PBS deficient
- Waschpuffer B:: 0,05 % Tween 20 in PBS deficient
- Blockierungspuffer A:: 5 % foetales Kälberserum (hitzeinaktiviert)
in PBS deficient
- Blockierungspuffer B:: 1 % Rinderserumalbumin
0,1 % NaN₃
in PBS deficient
- Verdünnungspuffer A:: 2% foetales Kälberserum (hitzeinaktiviert)
in PBS deficient
- Verdünnungspuffer B:: PBS deficient
- Färbepuffer:: 24,3 mM Zitronensäure
51,4 mM Na₂HPO₄
pH-Wert: 5,0
- Substrat:: 40 mg o-Phenylendiamin Dihydrochlorid
100 ml Färbepuffer
20 µl H₂O₂ (30%)
- Stopplösung:: 4 N H₂SO₄
- Formulierungspuffer:: 10 % PBS def, pH = 6,0
90 % physiologische NaCl-Lösung

### Beispiel 1

### Herstellung einer Immuno-Affinitäts-Säule mit einem TAA-spezifischen Antikörper

7,5 g CH-Sepharose 4B (Pharmacia) wurden für 15 Minuten in 20 ml 1 mM HCl suspendiert. Das Gel wurde dann mit 1 Liter 1 mM HCl und anschließend mit 200 ml Kopplungspuffer auf einem Sinterglasfilter AG3 gewaschen. 100 mg muriner Antikörper 17-1A (Panorex, Stammlösung 10 mg/ml; gerichtet gegen das Tumor-assoziierte Antigen Ep-CAM) wurden gegen 5 Liter Kopplungspuffer dialysiert und mit Kopplungspuffer auf 5 mg/ml eingestellt. Diese Lösung wurde mit der Gelsuspension in einem verschlossenen Gefäß vermischt. Ein Verhältnis von Gel : Puffer von 1 : 2 ergibt eine für die Kopplung geeignete Suspension. Diese Suspension wurde 24 Stunden bei 4°C rotiert. Anschließend wurde der Überschuß des Liganden mit 3 x 30 ml Kopplungspuffer weggewaschen. Überbleibende reaktive Gruppen wurden durch einstündige Inkubation bei 4°C mit 1 M Äthanolamin geblockt. Dann wurde das Gel für eine Stunde bei Raumtemperatur mit einem 0,1 M Tris-HCl Puffer rotiert. Letztlich wurde das Gel mit 3 Zyklen von Puffern mit altemierendem pH gewaschen. Jeder Zyklus besteht aus 0,1 M Natriumacetat Puffer pH 4 mit 0,5 M NaCl, und anschließend 0,1 M Tris-HCl Puffer pH 8 mit 0,5 M NaCl. Das Gel wurde bei 4°C aufbewahrt.

### Beispiel 2

### Immunaffinitätsreinigung einer Antikörperfraktion aus Serum mittels Immun-Affinitätschromatographie

Rhesusaffen wurde 10 ml peripheres Blut abgenommen und daraus Serum gewonnen. Die ganze Prozedur wurde unter sterilen Bedingungen durchgeführt:

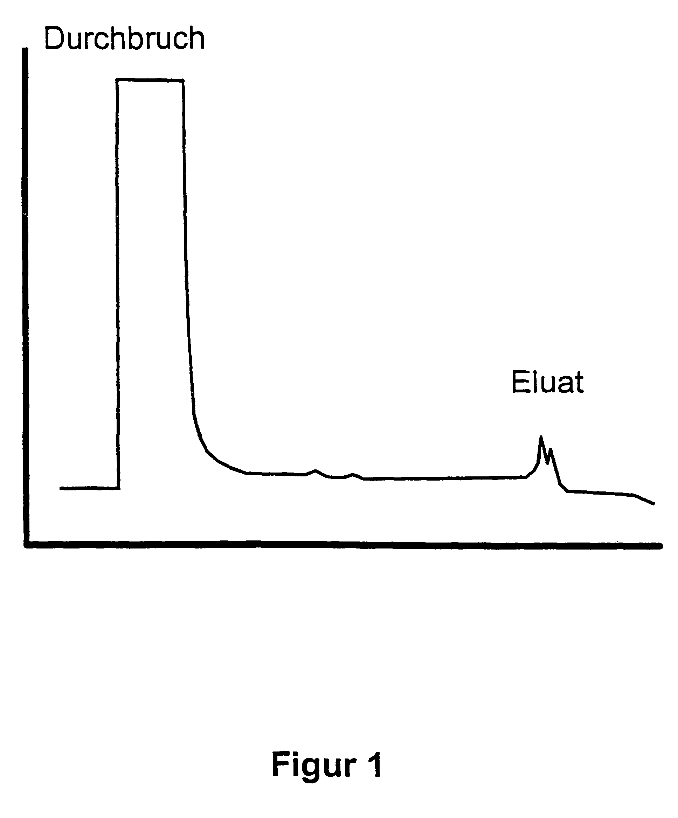

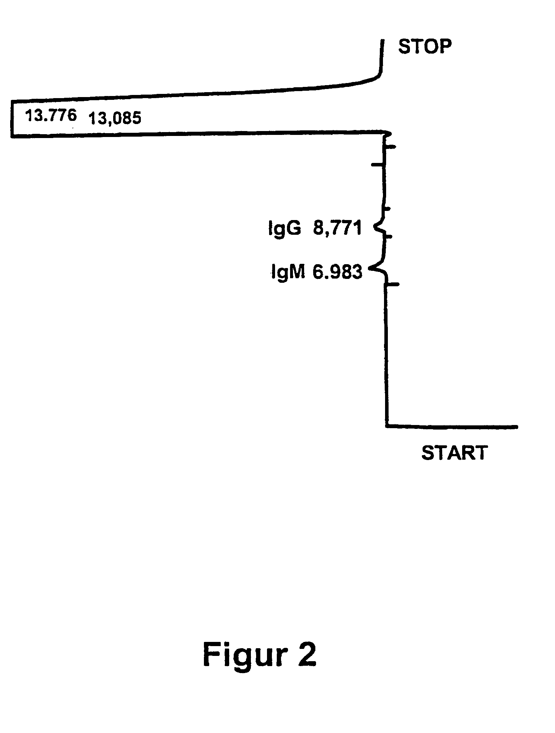

Die Immunaffinitätsreinigung wurde auf einem FPLC-System (Pharmacia) durchgeführt. 1 ml des nach Beispiel 1 erhaltenen Gels wurde in eine Pharmacia HR5/5 Säule gefüllt. 5 ml Serum wurden 1:10 mit dem Reinigungspuffer A verdünnt. Diese Lösung wurde mit 1 ml / Minute über die Säule gepumpt und mit Reinigungspuffer A nachgewaschen, bis die UV-Basislinie des Detektors wieder erreicht ist (280 nm). Gebundene Immunglobuline wurden dann mit Reinigungspuffer B eluiert und die Fraktion mit 1 M Na₂HPO₄ unmittelbar nach Desorption neutralisiert. Ein Chromatogramm dieser Reinigung (UV 280 nm) findet sich in Figur 1.
50µl der so gereinigten Antikörperfraktion wurden auf einer Größentrennungssäule (SEC, Zorbax 250 GF) analysiert. Als Laufmittel wurde 220 mM Phosphatpuffer pH 7 + 10% Acetonitril verwendet. Wie aus dem Chromatogramm dieser Analyse ersichtlich ist (Figur 2), beinhaltet die Antikörperfraktion IgM und IgG im Verhältnis von ca. 3:2. Die Gesamtmenge der Antikörperfraktion betrug ca 40 µg (bestimmt mittels SEC im Vergleich zu einem Standard).

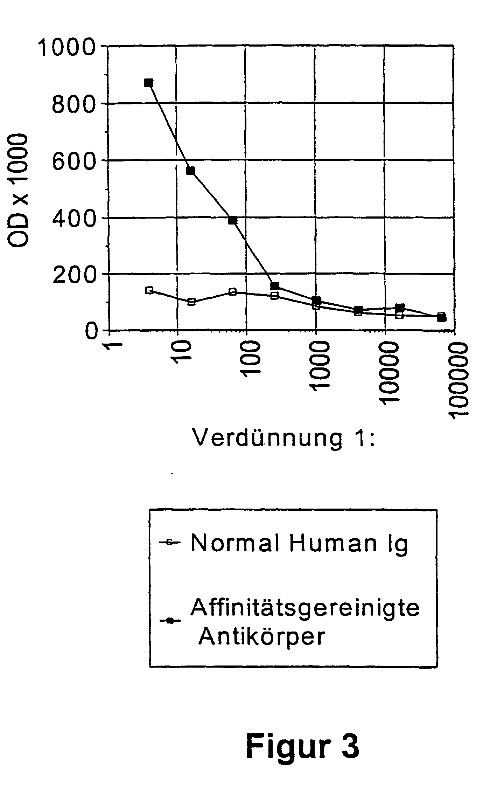

Die so gewonnene Antikörperfraktion wurde in einem ELISA bezüglich der Bindung an Antikörper 17-1A (der zur Affinitätsreinigung als Ligand verwendet wurde) getestet:
100 µl Aliquots des für die Affinitätsreinigung eingesetzten Antikörpers gegen ein Tumor-assoziiertes Antigen (Antikörper 17-1A; Lösung mit 10µg/ml in Bindungspuffer) wurden in den Näpfchen einer Mikrotiterplatte 1 Stunde bei 37°C inkubiert. Nach sechsmaligem Waschen der Platte mit Waschpuffer A wurden je 200µl des Blockierungspuffers A zugesetzt und 30 Minuten bei 37°C inkubiert. Nach Waschen der Platte wie oben beschrieben wurden je 100µl Aliquots der zu testenden affinitätsgereinigten Antikörperfraktion sowie normales Humanimmunglobulin in gleicher Konzentration als Negativkontrolle in Verdünnungen von 1:4 bis 1:65 000 in Verdünnungspuffer A 1 Stunde bei 37°C inkubiert. Nach Waschen der Platte wie oben beschrieben wurden je 100µl des Peroxidase-konjugierten Ziegen-anti-Human-Ig Antikörpers (Zymed) in einer Verdünnung von 1:1000 in Verdünnungspuffer A zugesetzt und 30 Minuten bei 37°C inkubiert. Die Platte wurde viermal mit Waschpuffer A und zweimal mit Färbepuffer gewaschen. Die Antikörperbindung wurde durch Zusatz von je 100µl des spezifischen Substrats nachgewiesen und die Farbreaktion durch Zugabe von je 50µl Stopplösung nach ca. 3 Minuten abgebrochen. Die Auswertung erfolgte durch Messen der optischen Dichte (OD) bei 490nm (Wellenlänge der Referenzmessung ist 620nm).
Wie in Figur 3 ersichtlich ist, zeigt die affinitätsgereinigte Antikörperfraktion eine deutliche Bindung an den Antikörper 17-1A, während normales Human-Immunglobulin praktisch nicht bindet.

### Beispiel 3

### Formulierung eines Impfstoffes aus gereinigten Antikörpern

Die durch Affinitätsreinigung erhaltene Antikörperfraktion wurde mit Aluminium-hydroxid als Adjuvans nach folgender Vorschrift formuliert:
3 ml der nach Affinitätschromatographie erhaltenen Antikörperlösung (enthält ca 40 µg Antikörper) wurden mit 1 mg Aluminiumhydroxid (wässrige Suspension; Alhydrogel, Superfos) versetzt und die Suspension in einem "FILTRON" Zentrifugenröhrchen (Microsep ™, cut-off 10KD) bei 4000 x g 30 Minuten lang zentrifugiert. Anschließend wurde 2 x mit je 1ml des Formulierungspuffers aufgeschlämmt und bei 4000 x g 30 Minuten lang zentrifugiert. Die Suspension wurde mit Formulierungspuffer auf 0,5 ml aufgefüllt und die so erhaltene Suspension steril abgefüllt.

### Beispiel 4

### Immunisierung von Rhesusaffen mit autologen, immun-affinitätsgereinigten Antikörpern

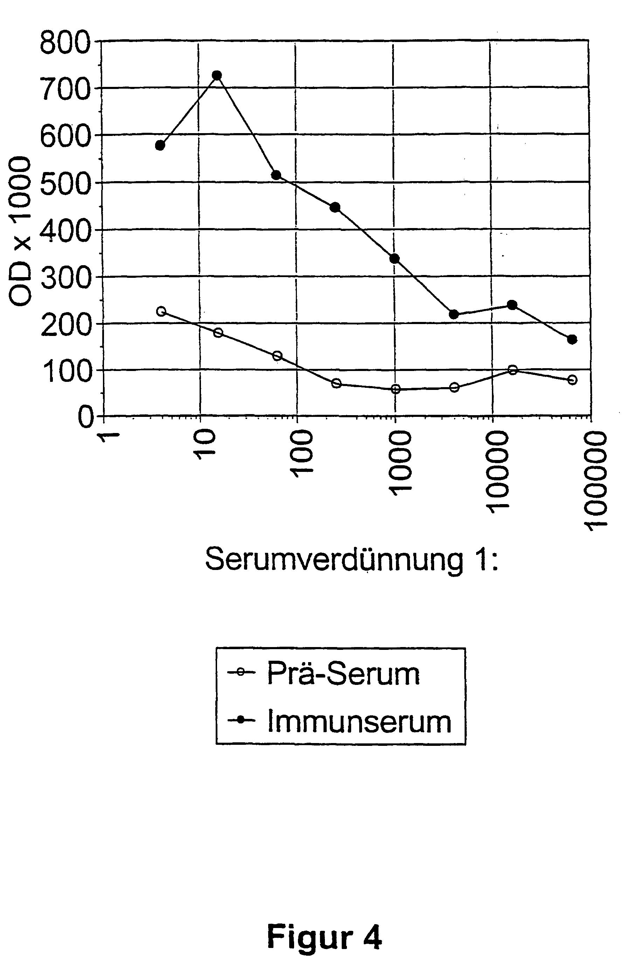

Der jeweilige Rhesusaffe, aus dessen Serum der Impfstoff wie in Beispiel 2 und 3 gewonnen und hergestellt wurde, wurde mit diesem Impfstoff subkutan in den Rücken geimpft. Vor dieser ersten Impfung wurden 5 ml Blut zur Serumgewinnung (zur Bestimmung des Ausgangswertes für die Charakterisierung der Immunantwort) abgenommen. Zwei Wochen danach wurden erneut 10 ml Blut zur Serumgewinnung abgenommen. Mit 4 ml dieses Serums wurde die Gewinnung eines autologen Impfstoffes wiederholt, wie oben beschrieben. Der so erneut gewonnene Impfstoff wurde dem Rhesusaffen wieder subkutan in den Rücken injiziert. 4 Wochen nach dieser Impfung wurden wieder 5 ml Blut zur Serumgewinnung abgenommen (zur Bestimmung des Effektes der beiden Impfungen).
Das Prä-Serum dieses Rhesusaffen und das Immunserum 4 Wochen nach der 2. Impfung wurde in einem Zell-ELISA auf Bindung von Antikörpern auf die Kato III Zellinie untersucht. Zu diesem Zweck wurde wie folgt vorgegangen:
Die Näpfchen einer Mikrotiterplatte wurden mit je 100 µl einer Zellsuspension der Kato III Zellinie in der Konzentration von 2x10⁶ Zellen/ml in Medium A über Nacht bei +37°C inkubiert. Nach Absaugen des Überstandes wurde die Platte mit je 50µl Fixierlösung pro Loch 5 Minuten bei Raumtemperatur inkubiert. Nach Absaugen des Überstandes wurden je 200µl Blockierungspuffer B zupipettiert und die Platte 1 Stunde bei Raumtemperatur inkubiert. Nach zweimaligem Waschen mit je 200µl Waschpuffer B wurden je 100µl Aliquots der zu testenden Affenseren in Verdünnungen von 1:4 bis 1:56 000 in Verdünnungspuffer B 1 Stunde bei 37°C inkubiert. Nach zweimaligem Waschen der Platte mit je 100 µl eiskaltem Waschpuffer B wurden je 100µl eines Peroxidase konjugierten Ziegen-anti-Human-Ig Antikörpers (Zymed) in einer Verdünnung von 1:1000 in Verdünnungspuffer A zugesetzt und 45 Minuten bei 37°C inkubiert. Die Platte wurde dreimal mit je 100 µl eiskaltem Waschpuffer B gewaschen. Die Antikörperbindung wurde durch Zusatz von je 100µl des spezifischen Substrats nachgewiesen und die Farbreaktion durch Zugabe von je 50µl Stopplösung nach ca. 5 Minuten abgebrochen. Die Auswertung erfolgte durch Messen der optischen Dichte (OD) bei 490nm (Wellenlänge der Referenzmessung ist 620nm).
Wie aus Figur 4 ersichtlich ist, befinden sich im Immunserum des autolog geimpften Rhesuaffen Immunglobuline, die auf Kato III Tumorzellen binden können, während im Prä-Serum desselben Tieres solche Antikörper kaum nachweisbar sind.

Auf Grund der Ergebnisse der oben beschriebenen Experimente ist beispielhaft gezeigt, daß die Vakzinierung mit individuellen autologen Antikörperfraktionen, die durch Immunaffinitätsreinigung an einem monoklonalen Antikörper gegen ein Tumor-assoziiertes Antigen erhalten wurden, eine humorale Immunantwort ausgelöst wurde, die auf menschlichen Tumorzellen, die dieses Tumor-assoziierte Antigen exprimieren, bindet.

### Beispiel 5:

### Herstellung einer Immun-Affinitäts-Säule mit zwei verschiedenen TAA-spezifischen Antikörpern

Ein monoklonaler Maus-Antikörper, der mit EpCAM reagiert und ein monoklonaler Maus-Antikörper, der mit Lewis Y reagiert, wurden jeweils an aktivierte CH Sepharose 4B (Pharmacia Biotech, Schweden) nach Standardprozeduren gekoppelt: 7,5 g CH Sepharose 4B wurden 15 Minuten in 20 ml 1 mM HCl suspendiert und gequollen. Das Gel wurde dann mit 1 Liter 1 mM HCl und anschließend mit 200 ml Kopplungspuffer auf einem Sinterglasfilter AG3 gewaschen. 100 mg muriner Antikörper (Stammlösung 10 mg/ml) wurden gegen 5 Liter Kopplungspuffer dialysiert und mit Kopplungspuffer auf 5 mg/ml eingestellt. Diese Lösung wurde mit der Gelsuspension in einem verschlossenen Gefäß vermischt. Ein Verhältnis von Gel : Puffer von 1 : 2 ergibt eine für die Kopplung geeignete Suspension. Diese Suspension wurde 24 Stunden bei 4°C rotiert. Anschließend wurde der Überschuß des Liganden mit 3 x 30 ml Kopplungspuffer weggewaschen. Überbleibende reaktive Gruppen wurden durch einstündige inkubation bei 4°C mit 1 M Äthanolamin geblockt. Dann wurde das Gel für eine Stunde bei Raumtemperatur mit einem 0,1 M Tris-HCl Puffer rotiert. Letztlich wurde das Gel mit 3 Zyklen von Puffern mit atternierendem pH gewaschen. Jeder Zyklus besteht aus 0,1 M Natriumacetat Puffer pH 4 mit 0,5 M NaCl, und anschließend 0,1 M Tris-HCl Puffer pH 8 mit 0,5 M NaCl. Das Gel wurde bei 4°C aufbewahrt
Gleiche Volumina der beiden Affinitätsmatrizes wurden gemischt und als Immunaffinitäts-Chromatographiesäule gepackt (System Äkta, Pharmacia, Schweden)

### Beispiel 6:

### Reinigung von Antikörpern aus Affenserum durch simultane Immunaffintätsreinigung mit zwei verschiedenen TAA spezifischen Antikörpern

Immunologisch naiven Rhesusaffen wurde jeweils ungefähr 20 ml Blut abgenommen, jeweils 9 ml Serum wurden mittels der in Beispiel 5 beschriebenen Immunaffinitätschromatographie-Säule gereinigt (System Äkta, Pharmacia, Schweden).
Auftragepuffer: Reinigungspuffer A
Elutionspuffer: Reigungspuffer B

Das Eluat wurde mit 0,5 M Natriumhydrogenphosphat-Lösung neutralisiert.

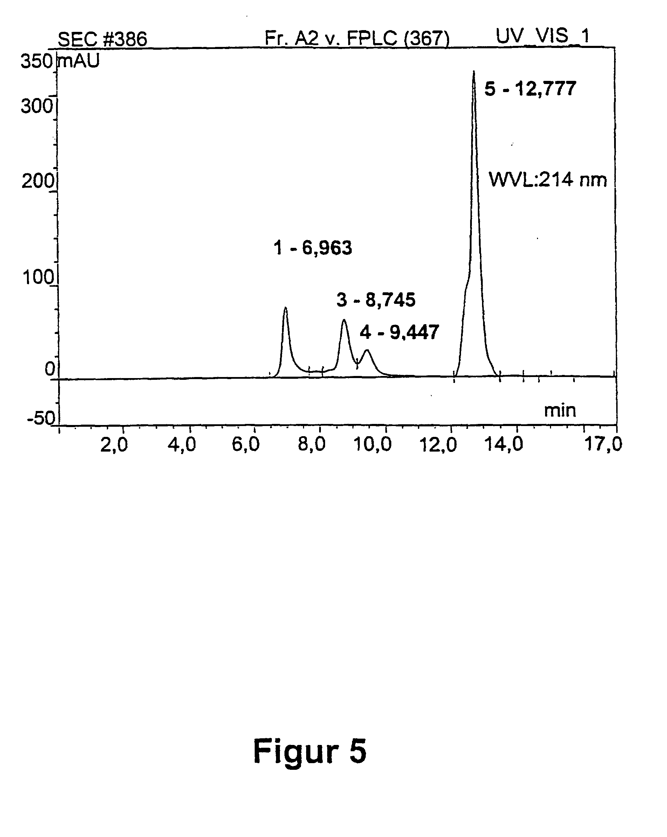

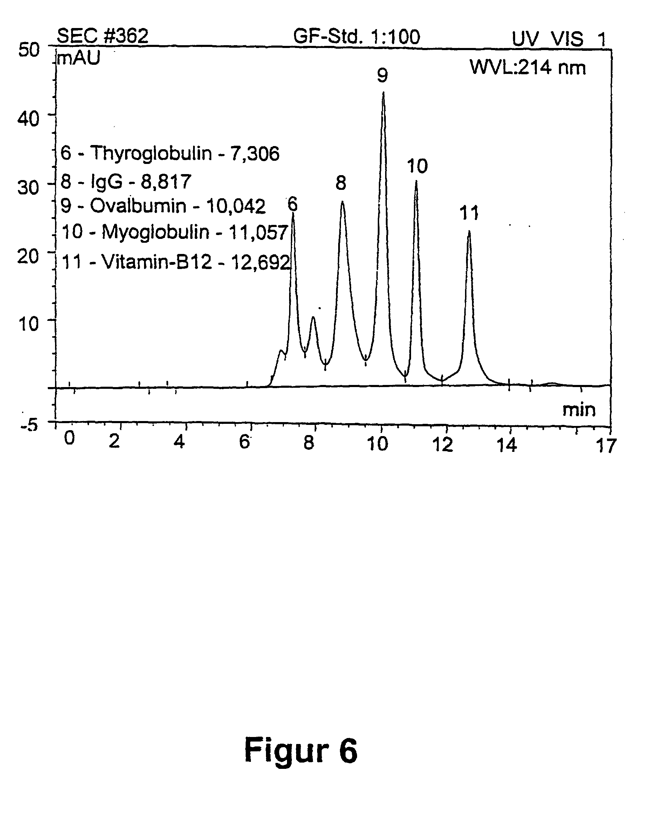

Die Eluate wurden derart fraktioniert, daß die gereinigten Proteine in hoher Konzentration vorliegen.
50µl der gereinigten Antikörperfraktion wurden auf einer Größentrennungssäule (SEC, Zorbax 250 GF) analysiert. Als Laufmittel wurde 220 mM Phosphatpuffer pH 7 + 10% Acetonitril verwendet (Flow: 1,000 ml/min; Wavelength 214 nm). Wie aus dem Chromatogramm dieser Analyse ersichtlich ist (Figur 5), beinhaltet die Antikörperfraktion IgM (Retentionszeit 6,963 min.) und IgG (Retentionszeit 8,745 min.) im Verhältnis von ca. 3:2. Die Gesamtmenge der Antikörperfraktion betrug ca. 50 µg (bestimmt mittels SEC im Vergleich zu einem Standard, Figur 6).

### Beispiel 7:

### Verschiedene Formulierungen eines Impfstoffes aus gereinigten Antikörpern

Der Impfstoff wurde in Ultrafiltrationseinheiten zum Zentrifugieren (Centricon 10; Amicon, USA) formuliert. Dazu wurden die Ultrafiltrationseinheiten mittels 1mM Na-Phosphatpuffer, 0,86% NaCl pH 6,0 (NBK) durch zentrifugieren gewaschen.
Danach wurde 1 ml Puffer (NBK) vorgelegt und 37µl Alhydrogel 2% (Superfos Biosector, Dänemark) zugegeben.
Nach Zugabe des neutralisierten Eluates aus Beispiel 6 wurde entsprechend der Centricon-Vorschrift zentrifugiert und gewaschen (mit 5ml Puffer).
Anschließend wurde die Vakzine resuspendiert, aufgefüllt auf 550µl mit Puffer und steril abgefüllt.
Alternativ wurde die Vakzine nach dem Resuspendieren mit Puffer auf 545µl aufgefüllt und mit 5,5µl Thimerosalstammlösung (10mg/ml, Sigma, USA) versetzt. Eine dritte Variante der Impfstoff-Formulierung wurde nach dem sterilen Abfüllen in Glasfläschchen in einem Autoklaven hitzedenaturiert (121°C; 20 min.).

## Patentansprüche

1. Verwendung von Antikörpern zur Herstellung einer pharmazeutischen Zusammensetzung geeignet als Impfstoff zur therapeutischen oder prophylaktischen Impfung gegen Krebs, **dadurch gekennzeichnet, dass** die Antikörper aus Antikörper enthaltenden Körperflüssigkeiten durch Immunaffinitätsreinigung gewonnen werden, wobei als Liganden für die Immunaffinitätsreinigung Antikörper verwendet werden, die ein oder mehrere Tumor-assoziierte Antigene erkennen, oder deren Fragmente mit gleichem Idiotyp, und wobei die Körperflüssigkeiten aus einem Individuum stammen, das nicht zuvor einen monoclonalen Antikörper verabreicht bekommen hat, der gegen eines der Tumor-assoziierten Antigene gerichtet ist.

2. Verwendung nach Anspruch 1, wobei die pharmazeutische Zusammensetzung bestimmt ist zur Verabreichung an das Individuum, aus dessen Körperflüssigkeit die Antikörper gewonnen wurden im Sinne eines autologen, individuellen Impfstoffes.

3. Verwendung nach Anspruch 1 oder 2, wobei die Körperflüssigkeit Serum ist.

4. Verwendung nach einem der Ansprüche 1 bis 3, wobei das Individuum ein Mensch ist.

5. Verwendung nach einem der Ansprüche 1 bis 4, wobei für die Immunaffinitätsreinigung gleichzeitig mehrere Antikörper gegen verschiedene Tumor-assoziierte Antigene und/oder verschiedene Epitope solcher Antigene verwendet werden.

6. Verwendung nach einem der Ansprüche 1 bis 5, wobei die als Liganden für die Immunaffinitätsreinigung verwendeten Antikörper monoclonale Antikörper sind.

7. Verwendung nach einem der Ansprüche 1 bis 6, wobei die pharmazeutische Zusammensetzung wiederholt hergestellt wird und bestimmt ist für die nachfolgende, in geeigneten Abständen wiederholte Verabreichung als individueller Impfstoff.

8. Verwendung nach einem der Ansprüche 1 bis 7, wobei es sich bei den Tumor-assoziierten Antigenen um häufig exprimierte und/oder co-exprimierte membranständige Moleküle auf Krebszellen epithelialen Ursprunges oder auf Krebszellen neuro-endokrinen Ursprunges oder auf Krebszellen des blutbildenden Systems handelt.

9. Verwendung nach einem der Ansprüche 1 bis 8, wobei die immunaffinitätsgereinigten Antikörper oder deren Derivate zusammen mit einem geeigneten Vakzineadjuvans formuliert werden.

10. Verwendung nach einem der Ansprüche 1 bis 9, wobei die Zusammensetzung weiterhin ein Protein enthält, das die Immunantwort verstärkt und das gleichzeitig mit den Antikörpern verabreicht wird.

11. Verwendung nach Anspruch 10, wobei das Protein ein humanes Protein ist.

12. Verwendung nach Anspruch 11, wobei das humane Protein Granulozyten-Makrophagen stimulierender Faktor (GM-CSF) ist.

13. Verwendung nach einem der Ansprüche 1 bis 12, wobei die Zusammensetzung geeignet ist für die Verabreichung der immunaffinitätsgereinigten Antikörper als Impfstoff durch subkutane oder intramuskuläre Injektionen.

14. Verwendung nach einem der Ansprüche 1 bis 13, wobei die in der Zusammensetzung enthaltenen Antikörper mit einem Adjuvans gemischt und einer Hitzebehandlung unterworfen werden.

15. Verwendung von isolierten ex-vivo kultivierten dendritischen Zellen eines Individuums zur Herstellung einer pharmazeutischen Zusammensetzung geeignet als Impfstoff zur therapeutischen oder prophylaktischen Impfung gegen Krebs, wobei die dendritischen Zellen mit Antikörpern zuvor in-vitro inkubiert wurden, welche aus einer Antikörper enthaltenden Körperflüssigkeit desselben Individuums durch Immunaffinitätsreinigung gewonnen wurden, wobei als Liganden für die Immunaffinitätsreinigung Antikörper verwendet werden, die ein oder mehrere Tumor-assoziierte Antigene erkennen, oder deren Fragmente mit gleichem Idiotyp, und wobei die Körperflüssigkeit aus einem Individuum stammt, das nicht zuvor einen monoclonalen Antikörper verabreicht bekommen hat, der gegen eines der Tumor-assoziierten Antigene gerichtet ist.

16. Pharmazeutische Zusammensetzung enthaltend IgM und IgG Antikörper, die aus Antikörper enthaltenden Körperflüssigkeiten durch Immunaffinitätsreinigung gewonnen wurden, wobei als Liganden für die Immunaffinitätsreinigung Antikörper verwendet werden, die ein oder mehrere Tumor-assoziierte Antigene erkennen, oder deren Fragmente mit gleichem Idiotyp, und wobei die Körperflüssigkeiten aus einem Individuum stammen, das nicht zuvor einen monoclonalen Antikörper verabreicht bekommen hat, der gegen eines der Tumor-assoziierten Antigene gerichtet ist.

17. Pharmazeutische Zusammensetzung enthaltend isolierte, ex-vivo kultivierte dendritische Zellen eines Individuums, wobei die dendritischen Zellen zuvor mit Antikörpern in-vitro inkubiert wurden, welche aus einer Antikörper enthaltenden Körperflüssigkeit desselben Individuums durch Immunaffinitätsreinigung gewonnen wurden, wobei als Liganden für die Immunaffinitätsreinigung Antikörper verwendet werden, die ein oder mehrere Tumor-assoziierte Antigene erkennen, oder deren Fragmente mit gleichem Idiotyp, und wobei die Körperflüssigkeit aus einem Individuum stammt, das nicht zuvor einen monoclonalen Antikörper verabreicht bekommen hat, der gegen eines der Tumor-assoziierten Antigene gerichtet ist.

18. Verfahren zur Herstellung einer Antikörperpräparation **dadurch gekennzeichnet, dass** aus einer Antikörper enthaltenden Körperflüssigkeit durch Immunaffinitätsreinigung Antikörper gewonnen werden, wobei als Liganden für die Immunaffinitätsreinigung Antikörper verwendet werden, die ein oder mehrere Tumor-assoziierte Antigene erkennen, oder deren Fragmente mit gleichem Idiotyp, und wobei die Körperflüssigkeit aus einem Individuum stammt, das nicht zuvor einen monoclonalen Antikörper verabreicht bekommen hat, der gegen eines der Tumor-assoziierten Antigene gerichtet ist.

19. Verfahren nach Anspruch 18, wobei für die Affinitätsreinigung gleichzeitig mehrere Antikörper gegen verschiedene Tumor-assoziierte Antigene und/oder Epitope solcher Antigene verwendet werden.

20. Verfahren nach Anspruch 18 oder 19, wobei die für die Affinitätsreinigung verwendeten Antikörper monoclonale Antikörper sind.

21. Verfahren nach einem der Ansprüche 18 bis 20, wobei die Körperflüssigkeit Serum ist.

22. Verfahren zur Herstellung einer pharmazeutischen Zusammensetzung, **dadurch gekennzeichnet, dass** eine Antikörperpräparation nach einem Verfahren nach einem der Ansprüche 18 bis 21 hergestellt wird und die so erhaltene Antikörperpräparation anschließend mit einem pharmazeutisch verträglichen Träger und/oder einem geeigneten Vakzineadjuvans formuliert wird.

23. Verfahren nach Anspruch 22, wobei die Antikörperpräparation mit einem Adjuvans gemischt und anschließend einer Hitzebehandlung unterworfen wird.

24. Verfahren nach einem der Ansprüche 18 bis 21, wobei die Immunaffinitätsreinigung der einzige Reinigungsschritt ist.

## Claims

1. Use of antibodies for the production of a pharmaceutical composition which is suitable as a vaccine for the therapeutic or prophylactic vaccination against cancer **characterised in that** the antibodies are recovered from body fluids containing antibodies by immunoaffinity purification, wherein antibodies recognising one or more tumour-associated antigens or fragments thereof having the same idiotype are used as ligands for the immunoaffinity purification and wherein the body fluids are derived from an individual that has not been administered a monoclonal antibody before which is directed against one of the tumour-associated antigens.

2. The use according to claim 1, wherein the pharmaceutical composition is intended for administration to the individual from the body fluid of which the antibodies were obtained in the sense of an autologous, individual vaccine.

3. The use according to claim 1 or 2, wherein the body fluid is serum.

4. The use according to any one of claims 1 to 3, wherein the individual is a human.

5. The use according to any one of claims 1 to 4, wherein several antibodies directed against different tumour-associated antigens and/or against different epitopes of such antigens are used at the same time for the immunoaffinity purification.

6. The use according to any one of claims 1 to 5, wherein the antibodies used as ligands for the immunoaffinity purification are monoclonal antibodies.

7. The use according to any one of claims 1 to 6, wherein the pharmaceutical composition is prepared repeatedly and is intended for subsequent administration as an individual vaccine repeated in suitable intervals.

8. The use according to any one of claim 1 to 7, wherein the tumour-associated antigens are membrane-located molecules which are frequently expressed and/or co-expressed on cancer cells of epithelial origin or on cancer cells of neuroendocrine origin or on cancer cells of the blood-forming system.

9. The use according to any one of claim 1 to 8, wherein the antibodies purified by immunoaffinity purification or the derivatives thereof are formulated together with a suitable vaccine adjuvant.

10. The use according to any one of claims 1 to 9, wherein the composition furthermore contains a protein which enhances the immune response and which is administered at the same time as the antibodies.

11. The use according to claim 10, wherein the protein is a human protein.

12. The use according to claim 11, wherein the human protein is granulocyte-macrophage stimulating factor (GM-CSF).

13. The use according to any one of claims 1 to 12, wherein the composition is suitable for administration of the antibodies purified by immunoaffinity purification as a vaccine by way of subcutaneous or intramuscular injections.

14. The use according to any one of claims 1 to 13, wherein the antibodies contained in the composition are mixed with an adjuvant and subjected to a heat treatment.

15. Use of isolated, ex vivo cultivated dendritic cells of an individual for the production of a pharmaceutical composition which is suitable as a vaccine for the therapeutic or prophylactic vaccination against cancer, wherein the dendritic cells have been incubated before in vitro with antibodies which have been obtained from an antibody-containing body fluid of the same individual by means of immunoaffinity purification, wherein antibodies recognising one or more tumour-associated antigens or fragments thereof having the same idiotype are used as ligands for the immunoaffinity purification and wherein the body fluid is derived from an individual that has not been administered a monoclonal antibody before which is directed against one of the tumour-associated antigens.

16. A pharmaceutical composition containing IgM and IgG antibodies obtained from body fluids containing antibodies by means of immunoaffinity purification, wherein antibodies recognising one or more tumour-associated antigens or fragments thereof having the same idiotype are used as ligands for the immunoaffinity purification and wherein the body fluids are derived from an individual that has not been administered a monoclonal antibody before which is directed against one of the tumour-associated antigens.

17. A pharmaceutical composition containing isolated, ex vivo cultivated dendritic cells of an individual, wherein the dendritic cells have been incubated before in vitro with antibodies which have been obtained from an antibody-containing body fluid of the same individual by means of immunoaffinity purification, wherein antibodies recognising one or more tumour-associated antigens or fragments thereof having the same idiotype are used as ligands for the immunoaffinity purification and wherein the body fluid is derived from an individual that has not been administered a monoclonal antibody before which is directed against one of the tumour-associated antigens.

18. A method for the production of an antibody preparation **characterised in that** antibodies are recovered from a body fluid containing antibodies by immunoaffinity purification, wherein antibodies recognising one or more tumour-associated antigens or fragments thereof having the same idiotype are used as ligands for the immunoaffinity purification and wherein the body fluid is derived from an individual that has not been administered a monoclonal antibody before which is directed against one of the tumour-associated antigens.

19. The method according to claim 18, wherein several antibodies directed against different tumour-associated antigens and/or epitopes of such antigens are used at the same time for the affinity purification.

20. The method according to claim 18 or 19, wherein the antibodies used for the affinity purification are monoclonal antibodies.

21. The method according to any one of claims 18 to 20, wherein the body fluid is serum.

22. A method for the production of a pharmaceutical composition **characterised in that** an antibody preparation is prepared in accordance with a method according to any one of claims 18 to 21 and the antibody preparation obtained in this way is subsequently formulated with a pharmaceutically acceptable carrier and/or a suitable vaccine adjuvant.

23. The method according to claim 22, wherein the antibody preparation is mixed with an adjuvant and, subsequently, subjected to a heat treatment.

24. The method according to any one of claims 18 to 21, wherein the immunoaffinity purification is the only purification step.

## Revendications

1. Utilisation d'anticorps pour la fabrication d'une composition pharmaceutique appropriée en tant que vaccin pour la vaccination thérapeutique ou prophylactique contre le cancer, **caractérisée en ce que** les anticorps sont extraits par purification par immuno-affinité des liquides de l'organisme comprenant des anticorps, des anticorps étant utilisés comme ligands pour la purification par immuno-affinité, anticorps qui reconnaissent un ou plusieurs antigènes associés à un tumeur ou leurs fragments au même idiotype, et les liquides de l'organisme provenant d'un individu auquel n'a pas été administré au préalable d'anticorps monoclonal qui est dirigé contre un des antigènes associés à un tumeur.

2. Utilisation selon la revendication 1, la composition pharmaceutique étant déterminée pour l'administration à l'individu, dont les anticorps ont été extraits du liquide de son organisme dans l'esprit d'un vaccin autologue et individuel.

3. Utilisation selon la revendication 1 ou 2, le liquide de l'organisme étant du sérum.

4. Utilisation selon l'une quelconque des revendications 1 à 3, l'individu étant un être humain.

5. Utilisation selon l'une quelconque des revendications 1 à 4, plusieurs anticorps, dirigés contre différents antigènes associés à un tumeur et/ou différents épitopes de tels antigènes, étant simultanément utilisés pour la purification par immuno-affinité.

6. Utilisation selon l'une quelconque des revendications 1 à 5, les anticorps utilisés comme ligands pour la purification par immuno-affinité étant des anticorps monoclonaux.

7. Utilisation selon l'une quelconque des revendications 1 à 6, la composition pharmaceutique étant fabriquée à plusieurs reprises et déterminée pour l'administration subséquente réitérée à intervalles appropriés en tant que vaccin individuel.

8. Utilisation selon l'une quelconque des revendications 1 à 7, sachant que pour les antigènes associés à un tumeur il s'agit de molécules membranaires souvent exprimées et/ou co-exprimées sur des cellules cancéreuses d'origine épithéliale ou sur des cellules cancéreuses d'origine neuroendocrinienne ou sur des cellules cancéreuses du système hématopoïétique.

9. Utilisation selon l'une quelconque des revendications 1 à 8, les anticorps purifiés par purification par immuno-affinité ou leurs dérivés étant formulés conjointement avec un adjuvant de vaccin approprié.

10. Utilisation selon l'une quelconque des revendications 1 à 9, la composition comprenant en outre une protéine qui renforce la réponse immunitaire et qui est administrée en même temps que les anticorps.

11. Utilisation selon la revendication 10, la protéine étant une protéine humaine.

12. Utilisation selon la revendication 11, la protéine humaine étant un facteur stimulant des macrophages granulocytes (GM-CSF).

13. Utilisation selon l'une quelconque des revendications 1 à 12, la composition étant appropriée pour l'administration des anticorps purifiés par purification par immuno-affinité en tant que vaccin par injections sous-cutanées ou intramusculaires.

14. Utilisation selon l'une quelconque des revendications 1 à 13, les anticorps compris dans la composition étant mélangés avec un adjuvant et soumis à un traitement thermique.

15. Utilisation de cellules dendritiques, isolées et cultivées ex-vivo, d'un individu pour la fabrication d'une composition pharmaceutique appropriée en tant que vaccin pour la vaccination thérapeutique ou prophylactique contre le cancer, l'incubation des cellules dendritiques ayant été au préalable opérée in vitro avec des anticorps qui ont été extraits par purification par immuno-affinité d'un liquide de l'organisme comprenant des anticorps du même individu, des anticorps étant utilisés comme ligands pour la purification par immuno-affinité, anticorps qui reconnaissent un ou plusieurs antigènes associés à un tumeur ou leurs fragments au même idiotype, et le liquide de l'organisme provenant d'un individu auquel n'a pas été administré au préalable d'anticorps monoclonal qui est dirigé contre un des antigènes associés à un tumeur.

16. Composition pharmaceutique comprenant des anticorps IgM et IgG qui ont été extraits par purification par immuno-affinité des liquides de l'organisme comprenant des anticorps, des anticorps étant utilisés comme ligands pour la purification par immuno-affinité, anticorps qui reconnaissent un ou plusieurs antigènes associés à un tumeur ou leurs fragments au même idiotype, et les liquides de l'organisme provenant d'un individu auquel n'a pas été au préalable administré d'anticorps monoclonal qui est dirigé contre un des antigènes associés à un tumeur.

17. Composition pharmaceutique comprenant des cellules dendritiques, isolées et cultivées ex-vivo, d'un individu, une incubation des cellules dendritiques ayant été opérée au préalable in vitro avec des anticorps qui ont été extraits par purification par immuno-affinité d'un liquide de l'organisme du même individu comprenant des anticorps, des anticorps ayant été utilisés comme ligands pour la purification par immuno-affinité qui reconnaissent un ou plusieurs antigènes associés à un tumeur ou leurs fragments au même idiotype, et le liquide de l'organisme provenant d'un individu auquel n'a pas été administré au préalable d'anticorps monoclonal qui est dirigé contre un des antigènes associés à un tumeur.

18. Procédé pour la fabrication d'une préparation d'anticorps, **caractérisée en ce que** des anticorps sont extraits par purification par immuno-affinité d'un liquide de l'organisme comprenant des anticorps, des anticorps étant utilisés comme ligands pour la purification par immuno-affinité, anticorps qui reconnaissent un ou plusieurs antigènes associés à un tumeur ou leurs fragments au même idiotype, et le liquide de l'organisme provenant d'un individu auquel n'a pas été administré au préalable d'anticorps monoclonal qui est dirigé contre un des antigènes associés à un tumeur.

19. Procédé selon la revendication 18, plusieurs anticorps dirigés contre différents antigènes associés à un tumeur et/ou des épitopes de tels antigènes étant simultanément utilisés pour la purification par affinité.

20. Procédé selon la revendication 18 ou 19, les anticorps utilisés pour la purification par affinité étant des anticorps monoclonaux.

21. Procédé selon l'une quelconque des revendications 18 à 20, le liquide de l'organisme étant du sérum.

22. Procédé pour la fabrication d'une composition pharmaceutique, **caractérisé en ce qu'**une préparation d'anticorps est fabriquée selon un procédé selon l'une quelconque des revendications 18 à 21 et la préparation d'anticorps ainsi obtenue est ensuite formulée avec un porteur toléré du point de vue pharmaceutique et/ou un adjuvant de vaccin approprié.

23. Procédé selon la revendication 22, la préparation d'anticorps étant mélangée à un adjuvant et ensuite soumise à un traitement thermique.

24. Procédé selon l'une quelconque des revendications 18 à 21, la purification par immuno-affinité étant la seule étape de purification.
